# FASCICULE DE BREVET EUROPEEN

(11) **EP 4 233 730 B1**
(45) Date de publication et mention de la délivrance du brevet: **12.03.2025**
(21) Numéro de dépôt: 23172405.5
(22) Date de dépôt: 01.06.2016
(51) Int. Cl.: A61B 5/00, A61B 8/08

(54) **DISPOSITIF NON INVASIF DE DÉTECTION DE LÉSION HÉPATIQUE**
NICHTINVASIVE VORRICHTUNG ZUR ERKENNUNG VON LEBERSCHÄDEN
NON-INVASIVE DEVICE FOR DETECTING LIVER DAMAGE

(30) Priorité: 02.06.2015 FR 1554995
(43) Date de publication de la demande: 30.08.2023
(62) Demande divisionnaire de: 16727453.9
(73) Titulaire: Echosens, 75014 Paris (FR)
(72) Inventeur: MIETTE, Véronique, 94800 Villejuif (FR); SANDRIN, Laurent, 94240 L'Hay les Roses (FR); SASSO, Magali, 30127 Bellegarde (FR)
(74) Mandataire: Cabinet Camus Lebkiri

(56) Documents cités:
- WO-A1-2013/025798
- WO-A2-2015/014763
- MIETTE V ET AL: "P1C-2 Liver Stiffness Measurement Using Transient Elastography in Patients with Non-Alcoholic Fatty Liver (NAFLD) and Non-Alcoholic Steatohepatitis (NASH)", ULTRASONICS SYMPOSIUM, 2007. IEEE, IEEE, PISCATAWAY, NJ, USA, 1 October 2007 (2007-10-01), pages 1333 - 1336, XP031195226, ISBN: 978-1-4244-1383-6, DOI: 10.1109/ULTSYM.2007.335
- MAGALI SASSO ET AL: "Controlled Attenuation Parameter (CAP): A Novel VCTE™ Guided Ultrasonic Attenuation Measurement for the Evaluation of Hepatic Steatosis: Preliminary Study and Validation in a Cohort of Patients with Chronic Liver Disease from Various Causes", ULTRASOUND IN MEDICINE AND BIOLOGY., vol. 36, no. 11, 1 November 2010 (2010-11-01), US, pages 1825 - 1835, XP055365057, ISSN: 0301-5629, DOI: 10.1016/j.ultrasmedbio.2010.07.005

## Description

### DOMAINE TECHNIQUE

L'invention concerne un dispositif non invasif de détection de lésion hépatique utilisant les ondes ultrasonores et les ondes de cisaillement. Ce dispositif peut être utilisé pour l'homme et pour l'animal et est par exemple destiné à la détection d'une lésion hépatique de type stéato-hépatite d'origine alcoolique (également connue sous l'acronyme ASH pour Alcoholic SteatoHepatitis en anglais) ou non alcoolique (également connue sous l'acronyme NASH pour Non Alcoholic Steato Hepatitis en anglais). L'invention permet de calculer un score reflétant une lésion hépatique.

### ART ANTERIEUR

Habituellement, les maladies chroniques du tissu hépatique génèrent des lésions hépatiques telles que la fibrose. La fibrose est un processus de cicatrisation fibreux du tissu hépatique résultant d'une inflammation. L'évolution de la fibrose initialement asymptomatique peut évoluer vers une cirrhose. L'élasticité du tissu hépatique constitue un marqueur de la fibrose hépatique. Afin de mesurer et quantifier l'élasticité du tissu hépatique, il est connu d'utiliser l'élastographie impulsionnelle, comme décrit, par exemple, dans la demande de brevet numéro FR 2843290.

Ce document présente une mise en œuvre d'un dispositif selon l'art antérieur. Ce dispositif est composé d'une sonde munie d'un générateur de vibration générant une onde élastique basse fréquence dans un tissu, par exemple par vibration, et analysant la propagation de cette onde élastique basse fréquence à l'aide d'ondes ultrasonores haute fréquence émises et reçues par un transducteur ultrasonore. Les mesures obtenues via ce dispositif permettent de quantifier l'élasticité du tissu hépatique. Ce dispositif permet également de quantifier l'atténuation ultrasonore des tissus, comme décrit, par exemple, dans la demande de brevet numéro FR 2949965. La quantification de l'atténuation ultrasonore dans le foie est corrélée la quantité de stéatose.

En revanche, chez l'homme certaines maladies, par exemple la NASH, ne sont pas nécessairement liées qu'à la seule quantité de fibrose ou à la seule quantité de stéatose, et peut par exemple associer des lésions de type stéatose (présence de graisse dans le foie) et inflammation avec ou sans fibrose. Par conséquent, le stade de NASH ou l'évolution vers la NASH ne peut pas être diagnostiqué au moyen d'un seul paramètre.

D'autres exemples d'art antérieur sont fournis par WO 2015/014763 A2 ; MIETTE V. ET AL. : « P1C-2 Liver Stiffness Measurement Using Transient Elastography in Patients with Non-Alcoholic Fatty Liver (NAFLD) and Non-Alcoholic Steatohepatitis (NASH) », IEEE ULTRASONICS SYMPOSIUM, 1 octobre 2007, pages 1333-1336 ; WO 2013/025798 A1 ; SASSO M. ET AL. : « Controlled Attenuation Parameter (CAP) : A Novel VCTE™ Guided Ultrasonic Attenuation Measurement for the Evaluation of Hepatic Steatosis: Preliminary Study and Validation in a Cohort of Patients with Chronic Liver Disease from Various Causes », ULTRASOUND IN MEDICINE AND BIOLOGY, NEW YORK, NY, US, vol. 36, no. 11, 1 novembre 2010, pages 1825-1835.

### EXPOSE DE L'INVENTION

La présente invention vise à résoudre au moins un des inconvénients de l'état de la technique précités. Pour cela, l'invention propose un dispositif selon la revendication 1.

En outre, la présente description a pour objet un dispositif de calcul d'un score pour l'homme ou l'animal ledit score étant une évaluation quantitative ou semi-quantitative d'une lésion hépatique de type stéato-hépatite d'origine alcoolique ou non-alcoolique, ledit dispositif de calcul étant construit et agencé pour calculer un score au moyen des paramètres au moins physiques voire biologiques suivants :
- Un paramètre corrélé à l'inflammation et/ou à la fibrose,
- Un paramètre corrélé à la stéatose.

Ce mode de réalisation présente notamment l'avantage de permettre une détection précoce de certains types de lésion hépatique, comme par exemple la NASH, la NASH pouvant être corrélée à une inflammation, à une fibrose et à une stéatose. En revanche, la NAFLD (pour Non-Alcoholic Fatty Liver Disease) est corrélée simplement à la stéatose. Grâce au score, il est donc possible de différencier des patients atteints d'une maladie de type NAFLD des patients atteints d'une maladie de type NASH.

Le score peut être une évaluation quantitative ou semi-quantitative (par exemple, indicateur binaire) d'une lésion hépatique de type stéato-hépatite d'origine alcoolique ou non alcoolique.

Le dispositif de calcul peut être embarqué dans :
- un échographe, ou
- un dispositif construit et agencé pour mesurer au moins l'élasticité hépatique.

Le dispositif peut être construit et agencé pour délivrer le score de manière concomitante aux paramètres physiques mesurés. En d'autres termes, l'échographe ou le dispositif construit et agencé pour mesurer au moins l'élasticité hépatique mesure des paramètres physiques et le dispositif selon l'invention calcule le score en prenant compte au moins des paramètres physiques mesurés.

Le dispositif de calcul peut être construit et agencé pour communiquer avec :
- un échographe distant, ou
- un dispositif distant construit et agencé pour mesurer au moins l'élasticité hépatique.

Selon l'invention, le paramètre corrélé à la fibrose est l'élasticité hépatique.

Selon l'invention, le paramètre corrélé à la stéatose peut être une mesure de l'atténuation des ondes ultrasonores, par exemple le paramètre appelé CAP tel que décrit dans l'article Sasso, M., et al. (2010). "Controlled attenuation parameter (CAP): a novel VCTE guided ultrasonic attenuation measurement for the evaluation of hepatic steatosis: preliminary study and validation in a cohort of patients with chronic liver disease from various causes." Ultrasound Med Biol 36(11): 1825-1835.

En alternative, selon l'invention, le paramètre corrélé à la stéatose peut être une mesure de viscosité du tissu hépatique.

Le dispositif de calcul peut être construit et agencé pour calculer un score au moyen d'au moins un paramètre supplémentaire corrélé au syndrome métabolique.

Le dispositif de calcul peut être construit et agencé pour calculer un score au moyen d'au moins un paramètre supplémentaire de type anthropomorphique.

Le dispositif de calcul peut être construit et agencé pour calculer un score au moyen d'au moins un paramètre supplémentaire de type biologique. Le au moins un paramètre biologique peut par exemple être choisi parmi les paramètres suivants : transaminases(ASAT, ALAT), GGT, PAL, Fer sérique, ferritine, saturation en transférine, adipokine (par exemple, adiponectine, leptine, resistine), cytokine (par exemple, TNFα, IL6, IL1-β), cholestérol HDL, glycémie, insulinémie, bilirubine, a2macroglobuline, haptoglobine, apolipoprotéine A1, CK18, triglycérides, adiponectine, urée, polymorphisme génétique (par exemple : polymorphisme PNPLA3, TM6SF2), CRP et/ou leptine.

Le dispositif de calcul peut être construit et agencé pour communiquer avec un dispositif d'affichage du score. Le score peut être affiché sous la forme d'une valeur numérique, d'un indicateur binaire, une probabilité ou un risque. Ce mode de réalisation présente notamment l'avantage de permettre une simplicité d'interprétation de l'analyse du score reflétant un état d'une liaison hépatique calculée.

La présente description porte également sur un score prenant en compte les paramètres au moins physiques voire biologiques suivants :
- Un paramètre corrélé à l'inflammation et/ou la fibrose, et
- Un paramètre corrélé à la stéatose.

Le score peut prendre en compte au moins un paramètre d'activité inflammatoire. Le au moins un paramètre d'activité inflammatoire peut être choisi parmi les paramètres suivants : la valeur des transaminases, l'élasticité hépatique ou la viscosité hépatique.

Le score peut prendre en compte au moins un paramètre anthropomorphique de type poids, taille, tour de taille, tour de hanche, périmètre thoracique ou un paramètre démographique de type âge et sexe.

Le score peut prendre en compte au moins paramètre biologique.

Le au moins un paramètre biologique peut être choisi parmi les paramètres suivants : transaminases (ASAT, ALAT), GGT, PAL, Fer sérique, ferritine, saturation en transférine, adipokine (par exemple, adiponectine, leptine, resistine) cytokine (par exemple, TNFα, IL6, IL1-β), cholestérol, cholestérol HDL, glycémie, insulinémie, bilirubine, a2macroglobuline, haptoglobine, apolipoprotéine A1, CK18, triglycérides, adiponectine, urée, polymorphisme génétique (par exemple : polymorphisme PNPLA3, TM6SF2), CRP et/ou leptine.

Le paramètre biologique peut être un paramètre métabolomique.

Le score est calculé au moyen de d'une modélisation statistique (également dénommé apprentissage statistique) de type régression logistique, arbres de décisions, classificateur bayésien, forêts aléatoires, SVM, réseau de neurone, analyse discriminante, etc.

### BREVE DESCRIPTION DES FIGURES

D'autres caractéristiques et avantages de l'invention ressortiront clairement de la description qui en est donnée ci-après, à titre indicatif et nullement limitatif, en référence :
- à la figure 1 illustrant, de façon schématique, un premier exemple de réalisation d'un dispositif de calcul d'un score reflétant un état d'une lésion hépatique intégré dans un dispositif construit et agencé pour mesurer l'élasticité hépatique,
- à la figure 2 illustrant, de façon schématique, un deuxième exemple de réalisation d'un dispositif de calcul d'un score reflétant un état d'une lésion hépatique construit et agencé pour communiquer avec un échographe distant.

### DESCRIPTION DE L'INVENTION

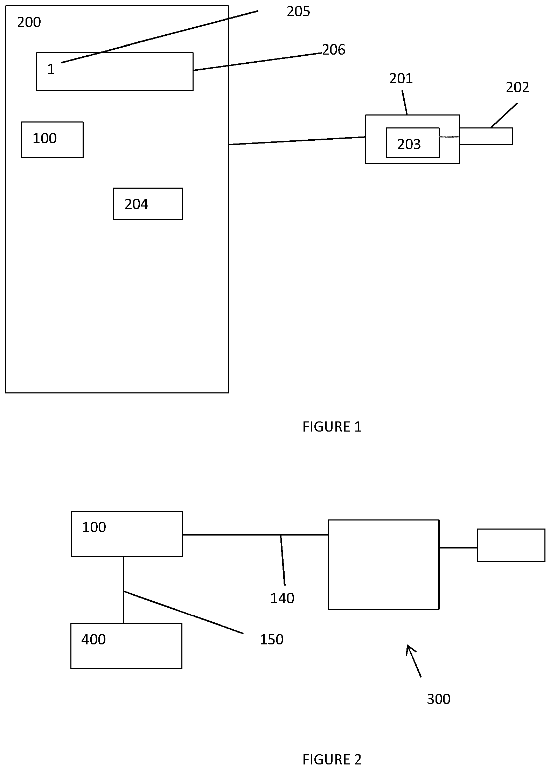

La figure 1 représente un dispositif 100 de calcul d'un score reflétant un état d'une lésion hépatique intégré dans un dispositif 200 construit et agencé pour mesurer l'élasticité hépatique.

Dans ce mode de réalisation non limitatif, le dispositif 200 comporte une sonde d'élastographie 201 munie d'un transducteur ultrasonore 202 construit et agencé pour émettre et recevoir des ondes ultrasonores. Dans ce mode de réalisation, la sonde d'élastographie 201 comporte en outre des moyens pour générer une onde de cisaillement dans le tissu hépatique. Lesdits moyens peuvent être un actionneur électrodynamique 203 construit et agencé pour générer une onde basse fréquence. Le dispositif 200 est donc construit et agencé pour mesurer des paramètres physiques, par exemple des paramètres qui sont corrélés à l'inflammation et/ou à la fibrose et des paramètres qui sont corrélés à la stéatose.

A titre d'exemple, un paramètre lié à la fibrose peut être l'élasticité du foie. Cette mesure d'élasticité constitue un marqueur de l'état pathologique du tissu hépatique.

Le paramètre corrélé à la stéatose peut être une mesure de l'atténuation des ondes ultrasonores dans le tissu hépatique. La stéatose hépatique est une accumulation de graisse dans le foie. La mesure de l'atténuation de la propagation des ondes ultrasonores permet donc de quantifier la stéatose.

Le dispositif 100 de calcul d'un score reflétant un état d'une lésion hépatique est construit et agencé pour calculer un score au moyen d'un paramètre corrélé à l'inflammation du tissu hépatique et/ou un paramètre corrélé à la fibrose. Dans l'exemple décrit, ces paramètres sont mesurés au moyen de la sonde d'élastographie 201.

Dans l'exemple illustré, le dispositif 200 comporte également une interface homme-machine 204 construite et agencée pour entrer des paramètres marqueurs du syndrome métabolique utilisés pour le calcul du score.

Ainsi, un opérateur peut entrer, via l'interface homme-machine 204, des paramètres marqueurs du syndrome métabolique. On entend par syndrome métabolique, l'association d'une série de problèmes de santé ayant en commun un mauvais métabolisme corporel, il s'agit d'un regroupement de facteurs de risque plus ou moins liés par une origine, des cibles métaboliques ou des mécanismes communs. Ce groupe de paramètres peut ainsi comporter : le cholestérol HDL, les triglycérides, la glycémie, la tension artérielle, et/ou le tour de taille.

Cette interface homme-machine 204 est également construite et agencée pour entrer des paramètres biologiques utilisés pour le calcul du score. Ces paramètres biologiques peuvent être : les transaminases (ALAT, ASAT), GGT, PAL, Fer sérique, cholestérol, cholestérol HDL, glycémie, insulinémie, bilirubine, a2macroglobuline, haptoglobine, apolipoprotéine A1, CK18, triglycérides, adiponectine, et/ou leptine.

Cette interface homme-machine 204 est également construite et agencée pour entrer des paramètres anthropomorphiques et démographiques utilisés pour le calcul du score. Ces paramètres démographiques et anthropomorphiques sont par exemple formés par l'âge, le sexe, la taille, le poids, le tour de taille, le tour de hanche ou le périmètre thoracique d'une personne.

En fonction de ces différents paramètres, le dispositif de calcul 100 calcule un score au moyen d'une régression logistique ou toute autre méthode de scoring, par exemple du type arbres de décisions, classificateur bayésien, forêts aléatoires, arbres de décision séparateurs à vaste marge (SVM), ou encore réseau de neurone.

A cette fin, le dispositif de calcul 100 peut être formé par un ou plusieurs microprocesseurs construit(s) et adapté(s) pour exécuter des séquences d'instructions permettant une mise en œuvre de la régression logistiques précitée ou toute autre méthode de scoring.

Dans l'exemple illustré, le score calculé est représenté sous la forme d'un indicateur binaire 205 égal à 1 et affiché sur un écran 206 du dispositif 200. Cet indicateur binaire 205 peut être utilisé pour recommander à un patient de réaliser une consultation auprès d'un spécialiste. Par exemple, lorsque l'indicateur est égal à 1, le patient est diagnostiqué comme étant à risque et nécessite une investigation plus poussée ou des examens complémentaires doivent être réalisés.

A contrario, lorsque l'indicateur est égal à 0, le patient ne doit pas consulter de spécialiste. Cet indicateur peut également être différent, il peut être implémenté sous la forme d'une valeur.

Dans cette réalisation non limitative, les mesures de paramètres physiques, l'entrée d'autres paramètres, le calcul du score et l'affichage du score sont réalisés sur le dispositif 200. Ainsi, ce mode de réalisation présente notamment l'avantage de calculer en temps réel le score (autrement dit sur le lieu où sont réalisées les mesures des paramètres physiques), puis d'afficher le score permettant ainsi une rapidité d'analyse.

Dans des exemples différents non limitatifs, le dispositif 200 peut être formé par un échographe, un IRM, ou un IRM mettant en œuvre l'élastographie par résonance magnétique (MRE).

Dans une mise en œuvre non limitative illustrée sur la figure 2, le dispositif de calcul d'un score reflétant un état d'une lésion hépatique 100 est construit et agencé pour communiquer avec un échographe 300 distant. Autrement-dit, le dispositif de calcul 100 est déporté vis-à-vis de l'échographe 300. Ainsi, les mesures sont réalisés sur l'échographe 300 puis transmises par une liaison réseau 140, par exemple une liaison de type Ethernet ou Bluetooth ou Wi-Fi, au dispositif de calcul 100. Il est également possible de transmettre d'autres paramètres, par exemple de type anthropomorphiques ou démographiques, au dispositif de calcul 100 via un ordinateur 400. De façon similaire, cet ordinateur 400 peut communiquer avec le dispositif de calcul 100 via une liaison Ethernet ou Wi-Fi 150. Le dispositif de calcul 100 peut être matérialisé par un ou plusieurs processeurs. Par ailleurs, l'ordinateur peut être intégré dans l'échographe 300.

Dans cette réalisation non limitative, l'affichage du score peut être réalisé sur l'échographe 300, sur l'ordinateur 400 ou les deux.

## Revendications

1. Dispositif de calcul (100) d'un score (205) pour l'homme ou l'animal, ledit score (205) étant une évaluation quantitative ou semi-quantitative d'une lésion hépatique de type stéato-hépatite d'origine alcoolique ou non-alcoolique, le score étant calculé au moyen d'une modélisation statistique, le score étant fonction des paramètres physiques et biologiques suivants :
- Un paramètre corrélé à la fibrose ;
- Un paramètre corrélé à la stéatose ; et
- Un paramètre corrélé à l'activité inflammatoire ;
dans lequel le paramètre corrélé à la fibrose est l'élasticité hépatique ;
dans lequel le paramètre corrélé à la stéatose est une mesure d'atténuation des ondes ultrasonores ou une mesure de viscosité hépatique ;
dans lequel le paramètre corrélé à l'activité inflammatoire est un paramètre parmi : ALAT, ASAT et GGT.

2. Dispositif de calcul (100) selon la revendication 1, dans lequel la modélisation statistique est une régression logistique, un arbre de décision, un classificateur bayésien, ou un réseau de neurones.

3. Dispositif de calcul (100) selon l'une des revendications précédentes, **caractérisé en ce qu'**il est embarqué dans :
- un échographe, ou
- un dispositif construit et agencé pour mesurer au moins l'élasticité hépatique.

4. Dispositif de calcul (100) selon l'une des revendications 1 et 2, **caractérisé en ce qu'**il est construit et agencé pour communiquer avec :
- un échographe, ou
- un dispositif construit et agencé pour mesurer au moins l'élasticité hépatique.

5. Dispositif de calcul (100) selon l'une quelconque des revendications précédentes, **caractérisé en ce qu'**il est construit et agencé pour calculer un score au moyen d'au moins un paramètre supplémentaire corrélé au syndrome métabolique.

6. Dispositif de calcul (100) selon l'une quelconque des revendications précédentes **caractérisé en ce qu'**il est construit et agencé pour calculer un score au moyen d'au moins un paramètre supplémentaire de type anthropomorphique.

7. Dispositif de calcul (100) selon l'une quelconque des revendications précédentes **caractérisé en ce qu'**il est construit et agencé pour calculer un score au moyen d'au moins un paramètre supplémentaire de type biologique.

8. Dispositif de calcul (100) selon l'une quelconque des revendications précédentes **caractérisé en ce qu'**il est construit et agencé pour communiquer avec un dispositif d'affichage (206) du score (205).

9. Dispositif de calcul (100) selon la revendication précédente **caractérisé en ce que** le score (205) est affiché sous la forme d'une valeur numérique, d'un indicateur binaire, une probabilité ou un risque.

## Patentansprüche

1. Vorrichtung (100) zur Berechnung eines Scores (205) für Mensch oder Tier, wobei der Score (205) eine quantitative oder semiquantitative Bewertung einer Leberschädigung vom Typ Steatohepatitis alkoholischen oder nichtalkoholischen Ursprungs ist, mittels statistischer Modellierung berechnet wird und von den folgenden physikalischen und biologischen Parametern abhängig ist:
- Ein mit Fibrose korrelierender Parameter;
- Ein mit Steatose korrelierender Parameter;
- Ein mit der Entzündungsaktivität korrelierender Parameter;
wobei der mit Fibrose korrelierende Parameter die Leberelastizität ist;
wobei der mit Steatose korrelierende Parameter eine Messung der Abschwächung von Ultraschallwellen bzw. eine Messung der Leberviskosität ist;
wobei der mit der Entzündungsaktivität korrelierende Parameter einer unter ALAT, ASAT und GGT ist.

2. Berechnungsvorrichtung (100) nach Anspruch 1, bei der die statistische Modellierung eine logistische Regression, ein Entscheidungsbaum, ein Bayes-Klassifikator oder ein neuronales Netz ist.

3. Berechnungsvorrichtung (100) nach einem der vorhergehenden Ansprüche, **dadurch gekennzeichnet, dass** sie eingebaut ist in:
- ein Ultraschallgerät oder
- einer Vorrichtung, die so ausgelegt und angeordnet ist, dass sie zumindest die Leberelastizität misst.

4. Berechnungsvorrichtung (100) nach einem der Ansprüche 1 oder 2, **dadurch gekennzeichnet, dass** sie so ausgelegt und angeordnet ist, dass sie kommuniziert mit:
- einem Ultraschallgerät oder
- einer Vorrichtung, die so ausgelegt und angeordnet ist, dass sie zumindest die Leberelastizität misst.

5. Berechnungsvorrichtung (100) nach einem der vorhergehenden Ansprüche, **dadurch gekennzeichnet, dass** sie so ausgelegt und angeordnet ist, dass sie einen Score mittels mindestens eines zusätzlichen Parameters, der mit dem metabolischen Syndrom korreliert, berechnet.

6. Berechnungsvorrichtung (100) nach einem der vorhergehenden Ansprüche, **dadurch gekennzeichnet, dass** sie so ausgelegt und angeordnet ist, dass sie einen Score mittels mindestens eines zusätzlichen Parameters anthropomorpher Art berechnet.

7. Berechnungsvorrichtung (100) nach einem der vorhergehenden Ansprüche, **dadurch gekennzeichnet, dass** sie so ausgelegt und angeordnet ist, dass sie einen Score mittels mindestens eines zusätzlichen Parameters biologischer Art berechnet.

8. Berechnungsvorrichtung (100) nach einem der vorhergehenden Ansprüche, **dadurch gekennzeichnet, dass** sie so ausgelegt und angeordnet ist, dass sie mit einer Anzeigevorrichtung (206) des Scores (205) kommuniziert.

9. Berechnungsvorrichtung (100) nach vorausgehendem Anspruch, **dadurch gekennzeichnet, dass** der Score (205) als numerischer Wert, als binärer Indikator für eine Wahrscheinlichkeit oder ein Risiko angezeigt wird.

## Claims

1. A device (100) for calculating a score (205) for human or animal, said score (205) being a quantitative or semi-quantitative evaluation of a liver injury of alcoholic or non-alcoholic steatohepatitis type, the score being calculated by means of statistical modelling, the score being a function of the following physical and biological parameters:
- a parameter correlated to fibrosis;
- a parameter correlated to steatosis; and
- a parameter correlated to inflammatory activity;
wherein the parameter correlated to fibrosis is liver elasticity;
wherein the parameter correlated to steatosis is an ultrasound wave attenuation measurement or a liver viscosity measurement;
wherein the parameter correlated to inflammatory activity is one of: ALAT, ASAT and GGT.

2. The calculation device (100) according to claim 1, wherein the statistical modelling is logistic regression, a decision tree, a Bayesian classifier or a neural network.

3. The calculation device (100) according to one of the preceding claims, **characterised in that** it is embedded in:
- an ultrasound scanner, or
- a device constructed and arranged to measure at least liver elasticity.

4. The calculation device (100) according to one of claims 1 and 2, **characterised in that** it is constructed and arranged to communicate with:
- an ultrasound scanner, or
- a device constructed and arranged to measure at least liver elasticity.

5. The calculation device (100) according to any of the preceding claims, **characterised in that** it is constructed and arranged to calculate a score by means of at least one additional parameter correlated to the metabolic syndrome.

6. The calculation device (100) according to any of the preceding claims, **characterised in that** it is constructed and arranged to calculate a score by means of at least one additional parameter of anthropomorphic type.

7. The calculation device (100) according to any of the preceding claims, **characterised in that** it is constructed and arranged to calculate a score by means of at least one additional parameter of biological type.

8. The calculation device (100) according to any of the preceding claims, **characterised in that** it is constructed and arranged to communicate with a device (206) for displaying the score (205).

9. The calculation device (100) according to the preceding claim, **characterised in that** the score (205) is displayed as a numerical value, a binary indicator, a probability or a risk.
